# EUROPEAN PATENT SPECIFICATION

(11) **EP 3 474 786 B1**
(45) Date of publication and mention of the grant of the patent: **03.03.2021**
(21) Application number: 17820643.9
(22) Date of filing: 16.06.2017
(51) Int. Cl.: A61F 2/50, A61F 2/60, A61F 2/68, A61F 2/76

(54) **SYSTEM AND ADAPTER DEVICE FOR FIXING A COVER TO A PROSTHETIC LIMB**
SYSTEM UND ADAPTERVORRICHTUNG ZUR BEFESTIGUNG EINER ABDECKUNG AN EINER GLIEDMASSENPROTHESE
SYSTÈME ET DISPOSITIF ADAPTATEUR POUR FIXER UN COUVERCLE À UN MEMBRE PROTHÉTIQUE

(30) Priority: 27.06.2016 SE 1650918
(43) Date of publication of application: 01.05.2019
(73) Proprietor: Anatomic Studios Sweden AB, 211 29 Malmö (SE)
(72) Inventor: DAHLBERG, Staffan, 211 21 Malmö (SE); FRANKSSON, Stephanie, 511 92 Örby (SE); NORDIN, Axel, 222 20 Lund (SE); ARLEMARK, Malkus, 216 13 Limhamn (SE)
(74) Representative: Neij & Lindberg AB
(86) International application number: PCT/SE2017/050651
(87) International publication number: WO 2018/004424

(56) References cited:
- WO-A1-2014/111263
- DE-A1-102010 049 894
- GB-A- 1 494 658
- US-A1- 2007 150 069
- US-A1- 2015 366 680
- Anonymous: "Product Info - Arthesis - English", , 28 May 2015 (2015-05-28), XP055659204, Retrieved from the Internet: URL:https://web.archive.org/web/2015052803 3323/http://www.arthesiscovers.com:80/en/p roductinfo [retrieved on 2020-01-17]

## Description

### TECHNICAL FIELD

The present disclosure relates to the field of prosthetics for limbs such as legs and arms. More particular the invention pertains to a prosthetic system adapted for fixing a cover to a prosthetic limb by using an adapter device.

### BACKGROUND ART

Today, around 6000 leg amputees live in Sweden and about 8,000 leg amputations are performed per year. Most get a prosthesis which they are happy with from a purely functional perspective, but the psychological aspect that comes when one loses a part of oneself is often ignored in the design of the prosthesis. A growing group of amputees, especially young and active, are asking for prostheses that are not reminiscent of a hospital setting and something they want to hide, but instead something that gives them more confidence, such as a prosthesis which rather looks like a fashion accessory than an aid.

Most prosthetic limb solutions are individually designed to gain optimal fitting and function. Cosmetic solutions will therefore also involve individual design to achieve best appearances with relative high costs as a result. Consequently most users are given no or few options to decide the cosmetic appearance of their prosthesis. This is partly due to costs involved with single-unit manufacturing. Recently, additive methods have been developed which has reduced the cost of prototyping and given greater freedom in the design language, but it is still an expensive process, and there are still requirements such as strength, weight, cosmetic fit with the user's healthy limb and prosthesis movement that need to be taken into consideration. Some of these problems can be facilitated by 3D scanning of the user's healthy limb and existing prosthesis, but it still requires many hours of work by orthopaedic engineers and designers, which greatly increases the cost to create a finished cosmetic solution that is ready for manufacturing.

GB 1494 658 relates to limb prostheses and particularly connections between the ends of first limb portions with cosmetic sheaths for connection to the body of the patient on the one hand and second limb portions such as foot or hand parts of skeletal prostheses on the other hand.

DE10 2010 049894 relates to a prosthesis device with a proximal joint device, a distal connecting element and a connecting element that connects the connecting element to the joint device, and a shell-like covering that at least partially covers the joint device and the connecting element while forming a cavity around the connecting element. The invention also relates to a covering as such for a prosthesis device.

"Product Info - Arthesis - English", 28 May 2015, XP055659204, retrieved from the Internet; URL:https://web.archive.org/web/20150528033323/http://www.arthesiscovers.com:80/en/pr oductinfo shows an arthesis cover with a back panel and a front panel, which is attached to the back panel by magnets.

US2015/366680 A1 relates to an intelligent prosthetic socket system with active user feedback interface and real time prosthesis diagnostics.

WO2014/111263 A1 relates to a prosthesis element for lining a prosthesis or for forming a prosthesis component and to a method for producing a prothesis element from a flat base material.

### SUMMARY OF THE INVENTION

An object of the present disclosure is to provide a system and device for fixing a cover to a prosthetic limb which seek to mitigate, alleviate, or eliminate one or more of the above-identified deficiencies in the art and disadvantages singly or in any combination.

The present disclosure is defined by the appended claims. Various advantageous embodiments of the disclosure are set forth by the appended claims as well as by the following description and the accompanying drawings.

The object is obtained by an adapter device according to claim 1 and a prosthesis system according to claim 9.

An advantage of the present disclosure is to offer persons with prostheses increased confidence and improved quality of life. A further advantage is to provide a safe and easy way of connecting and changing a cover to a prosthetic limb. The present disclosure further provides a solution for connecting the prosthetic system to externa communication devices. The price and lead time for custom-made cosmetic solutions for covers to prosthetic limbs are reduced dramatically and could ultimately lead to a commercially viable model.

### BRIEF DESCRIPTION OF THE DRAWINGS

The foregoing will be apparent from the following more particular description of the example embodiments, as illustrated in the accompanying drawings in which like reference characters refer to the same parts throughout the different views. The drawings are not necessarily to scale, emphasis instead being placed upon illustrating the example embodiments.
Figure 1 illustrates an example of a system according to the present disclosure;
Figure 2a illustrates an example of the adapter device according to the present disclosure;
Figure 2b illustrates an example of the adapter device according to the present disclosure;
Figure 3 illustrates an example of a first adapter part of the adapter device according to the present disclosure;
Figure 4a illustrates an example of a second adapter part of the adapter device according to the present disclosure;
Figure 4b illustrates an example of a second adapter part of the adapter device according to the present disclosure;
Figure 5a illustrates a cut-through view of the adapter device according to the present disclosure;
Figure 5b illustrates a cut-through view of the adapter device according to the present disclosure;
Figure 5c illustrates an example of a second adapter part of the adapter device according to the present disclosure;
Figure 5d illustrates an example of the adapter device according to the present disclosure;
Figure 5e-f illustrates a cut-through view of the adapter device according to the present disclosure;
Figure 5g illustrates an example of a second adapter part of the adapter device according to the present disclosure;
Figure 5h, illustrates an example of the adapter device according to the present disclosure;
Figure 6 illustrates an example of a system according to the present disclosure;
Figure 7 is a flow chart illustrating embodiments of method steps according to the present disclosure;
Figure 8 an example of the adapter device according to the present disclosure;

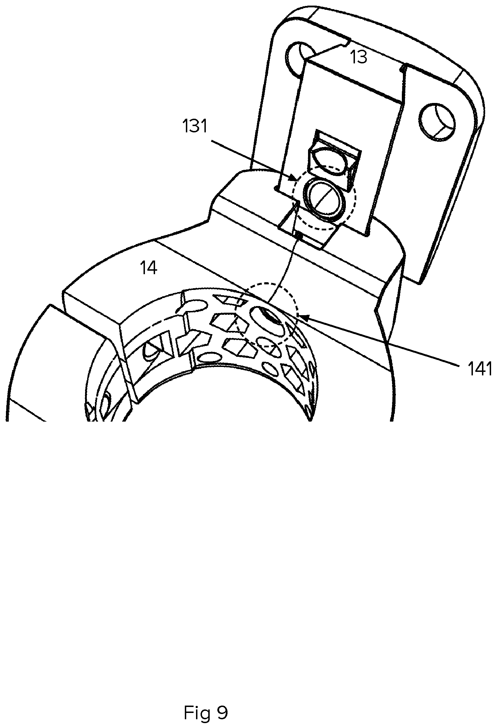

Figure 9 an example of the adapter device according to the present disclosure.
Figure 10 and example of the second adapter part according to the present disclosure.

### DETAILED DESCRIPTION

Aspects of the present disclosure will be described more fully hereinafter with reference to the accompanying drawings. The system, method and device disclosed herein can, however, be realized in many different forms and should not be construed as being limited to the aspects set forth herein. Like numbers in the drawings refer to like elements throughout.

The terminology used herein is for the purpose of describing particular aspects of the disclosure only, and is not intended to limit the disclosure. As used herein, the singular forms "a", "an" and "the" are intended to include the plural forms as well, unless the context clearly indicates otherwise.

Unless otherwise defined, all terms (including technical and scientific terms) used herein have the same meaning as commonly understood by one of ordinary skill in the art to which this disclosure belongs. It will be further understood that terms used herein should be interpreted as having a meaning that is consistent with their meaning in the context of this specification and the relevant art and will not be interpreted in an idealized or overly formal sense unless expressly so defined herein.

Some of the example embodiments presented herein are directed towards prosthetic covers. The disclosure relates to a prosthesis system adapted for being worn by a user comprising at least a first cover, a prosthetic limb and an adapter device for fixing the first cover to the prosthetic limb wherein the adapter device comprises a first adapter part configured for being attached to the first cover, a second adapter part configured for being mounted onto the prosthetic limb and locking means for locking the cover onto the prosthetic limb. As part of the development of the example embodiments presented herein, a problem will first be identified and discussed.

The prostheses available on the market today are mainly focused on function rather than aesthetics, aiming to provide physical quality of life for the user while the psychological aspects connected to use of prostheses are often given less attention. The possibilities of changing the prosthetic appearance as a way of expressing personality and style or depending on activity and situation are few.

The disclosed prosthetic system provides individually designed cosmetic covers to existing prosthetic limbs. The covers may be manufactured by using a 3D print process, e.g. Selective Laser Sintering, SLS. A solution including prosthetic covers with an adapter device for fixing the cover to the prosthetic limb that provides a quick and easy way of changing prosthetic covers when the user wishes. For example, the user may have a pair of covers that are striped with reflective film and are extremely light to be used when out running, and a more graceful colour matched cover to a dress to switch to when going to a party.

The uniqueness of the disclosed prosthetic system is to enable personalized prostheses at low cost by developing a software that automates parts of the design process, taking into account the technical requirements and helps the user to quickly and easily find a personal expression. This ability to adapt is unique not only in prosthetics, but in all consumer products. The only form of customization that is available today is that the customer can choose between prefabricated components in a modular system, or they can choose colours and materials. Some users may perform different physical activities, which means that the covers easily get bumps and they should not easily and unwillingly fall off. Thus it is an objective of the invention to provide a safe and stable way of fastening the cover to the prosthetic limb.

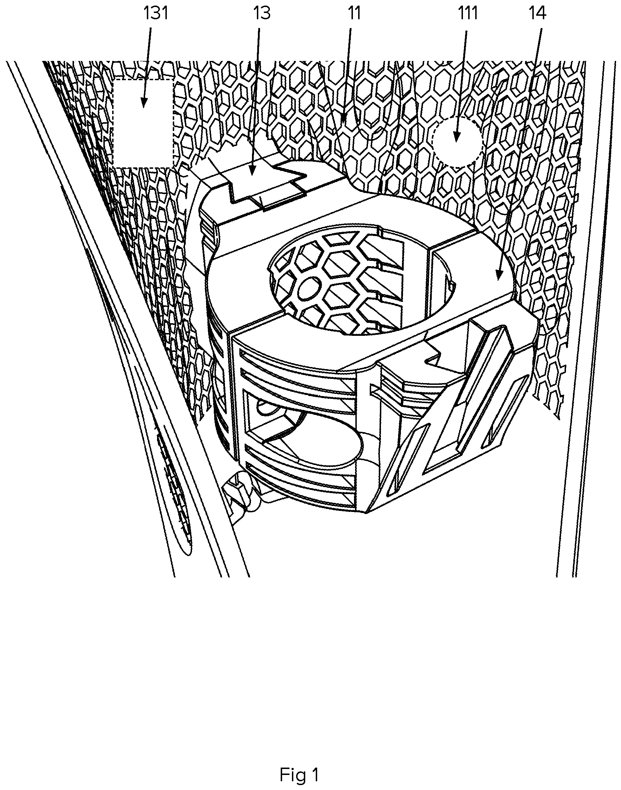

Figure 1 illustrates a prosthetic system 10 including an adapter device 13, 14 for fixing a cover 11 to a prosthetic limb 12. The adapter device comprises a first adapter part 13 configured for being attached to the cover, a second adapter part configured for being mounted onto the prosthetic limb and a locking means for locking the first adapter part onto the second adapter part when the first and second adapter parts are connected when fixing the cover to the prosthetic limb.

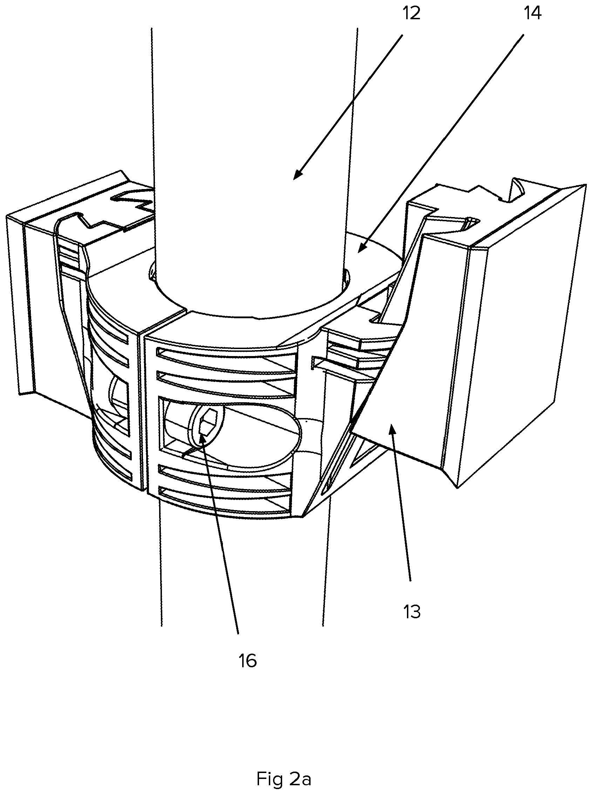

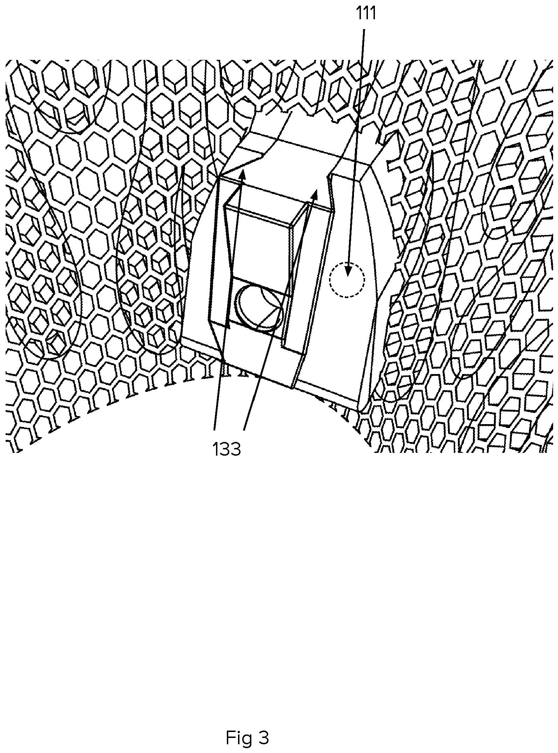

Figure 2a, illustrates an example of the basic geometry of the system after mounting with at least two fastening means 16 including e.g. a screws and low stop nut, against a tube of the prosthetic limb 12 of e.g. Aluminium. The same fastening means 16 is used on both sides. Figure 2a-b, 3, illustrates an example of a first adapter part 13 of the adapter device. This part may be injection moulded to keep the unit cost down and to obtain better strength. However, the part 13 may also be made by the same 3D print process as used for manufacturing the covers. In some aspect, the first adapter part 13 is manufactured together with the cover 11 as one unit. In some aspect, the first adapter part 13 is manufactured as a separate adapter part configured to be attached to the cover 11 before fixing the cover to the prosthetic limb.

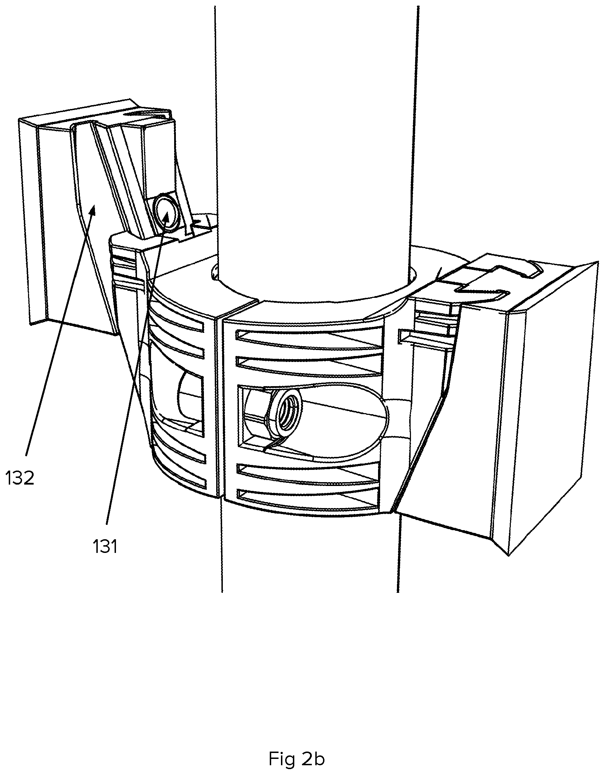

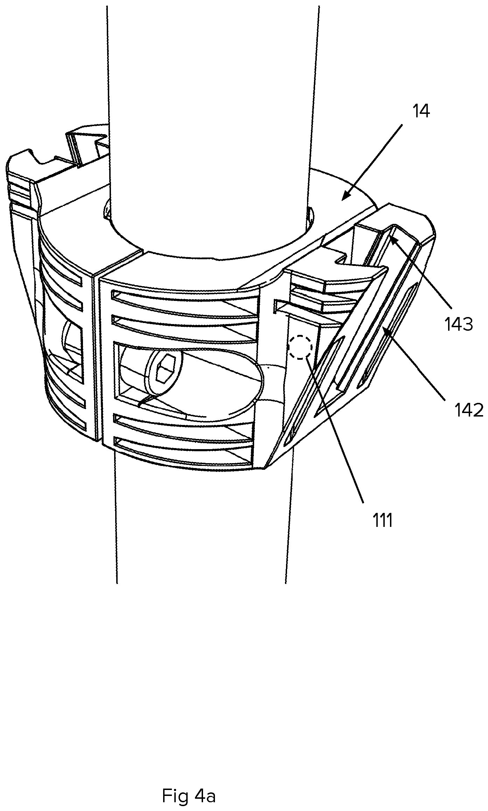

According to some aspect, a connecting surface 132, as shown in figure 2b, of the first adapter part 13 is inclined and a corresponding connecting surface 142, as shown in figure 4a, of the second adapter part 14 is inclined in the opposite direction according to the first adapter part 13. The inclines connecting surfaces provides an easy way of attaching or fixing the cover 11 to the prosthetic limb.

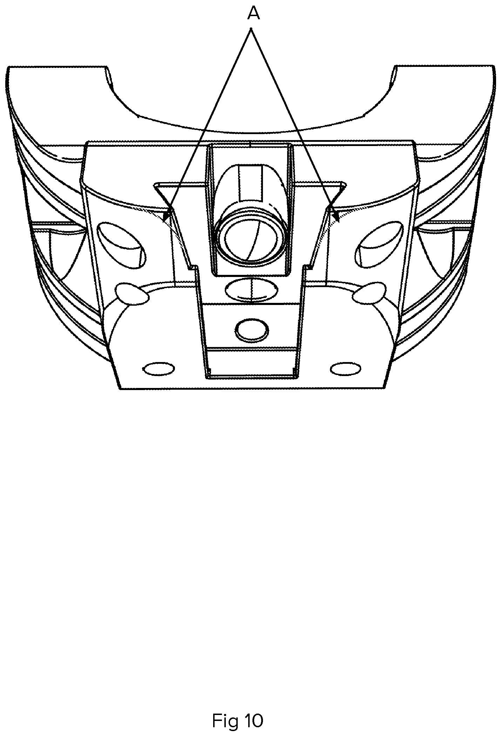

According to the invention the first adapter part 13 is provided with at least one protrusion 133, as shown in figure 3, and the second adapter parts is provided with at least one groove 143, as shown in figure 4a, for creating a dovetail connection when the first and second adapter parts are connected, as shown in figure 2a-b. The dovetail connection strengthens the connection between the first adapter part and the second adapter part. In one example, the protrusions are provided along at least two of the edges of the first adapter part 13 and the corresponding grooves are provided along at least two of the edges of the second adapter part 14. In one example, the groves edges are inclined A, as shown in figure 10, to minimize the damages on clothes and user when not wearing the cover 11.

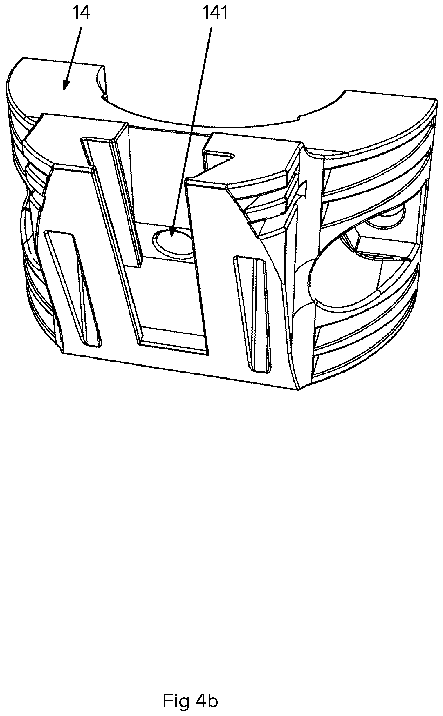

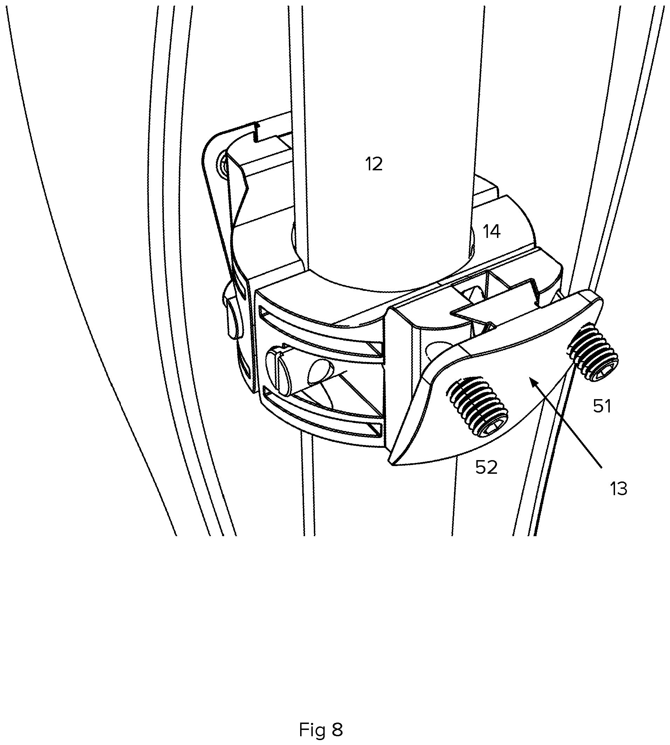

According to some aspects, the locking means comprises at least a first locking part 131, as shown in figure 2b, and a second locking part 141, as shown in figure 4b. The first locking part 131 is provided on the first adapter part 13 and the second locking part is provided on the second adapter part 14. In some aspect, the first locking part is a resilient pressing piece, e.g. a ball, and the second locking part is a hole adapted for collecting the resilient pressing piece when the first and second adapter parts are connected. In some aspects, the locking means comprises magnets adapted for locking the first and second adapter part when they are connected. In some aspect, as shown in figure 8, the first locking part 131 is a resilient pressing piece and the second locking part 141 is a resilient pressing piece and thus there is provided a double lock.

The principle is to provide an intuitive "click" such that the user knows when the cover is in the right position. Since the ball is connected to a spring, not shown, it is then resilient mounted. The ball will then always be under pressure and thus avoid chatter and noise that may otherwise be caused by vibrations when walking. The force of the spring may be pre-set to be able to determine how easy or hard the cover is fixed.

Figure 2a and 2b illustrates the principle of dovetail, where the first adapter part 13 slides down to the bottom of a grove or track provided in the second adapter part 14. Figure 2b illustrates where a resilient pressing piece, e.g. a ball, slides into a cavity 141, e.g. a hole, provided in the second adapter part 14, and locks the first adapter part 13 onto the second adapter part 14 and thus locks the parts together.

According to some aspects, as shown in figure 3 and 4a, one or both of the first and second adapter parts 13, 14 comprises a compartment adapted for enclosing or carrying a communication unit 111.

The first adapter part 13 may also act as a holder for e.g. a GoPro camera or other functional device etc.

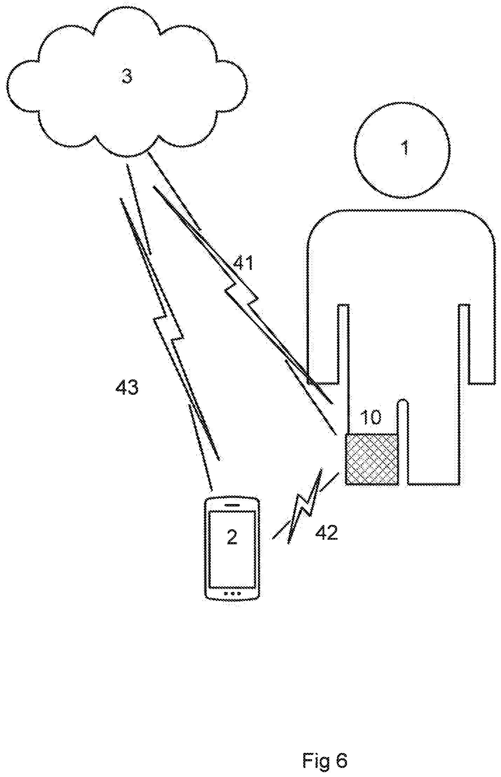

Figure 1 and 6 illustrates a prosthesis system 10 adapted for being worn by a user 1 comprising at least one cover 11 and a prosthetic limb 12. The system 10 further comprises the adapter device 13, 14 for fixing the cover 11 to the prosthetic limb 12. The adapter device comprises a first part 13 configured for being attached to the cover, a second part 14 configured for being mounted onto the prosthetic limb 12 and locking means 131, 141 for locking the first adapter part 13 onto the second adapter part 14 when the first and second adapter parts are connected.

The prosthetic limb 12 comprises a tube 121 and a prosthetic foot or hand.

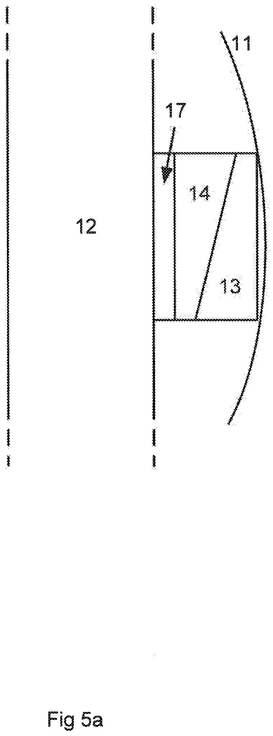

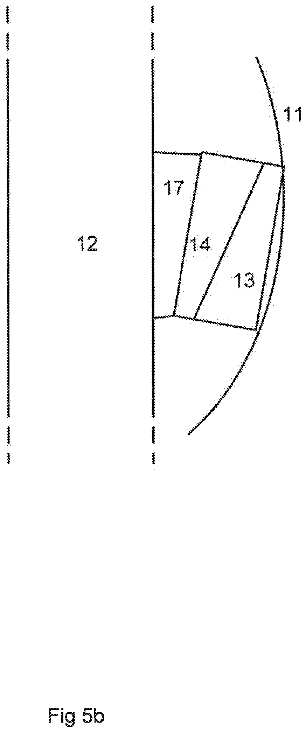

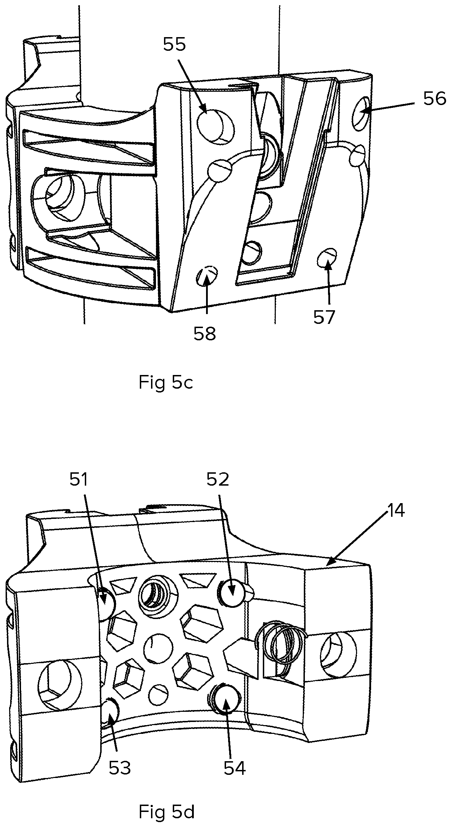

Figure 5a and 5b, shows derailment of material for applying a flexible piece 17 e.g. a rubber sheet between the adapter device 13, 14 and the tube of the prosthetic limb 12. The mission of the flexible piece 17 is to contribute to increased friction to prevent slippage, but also to adapt to different dimensions of the tube 121. The diameter of the tube between 30-34 mm is most common. The flexible piece may also be wedge-shaped, as shown in figure 5b, to be used to change the angle of the cover 11 if the user or prosthetist has changed the settings of the prosthesis, and it is necessary to compensate. The flexible piece also works as a cushion to prevent breakage on impact against the cover.

According to some aspects, the prosthesis system further comprises at least four screws 51, 52, 53, 54, with associated holes 55, 56, 57, 58, 59, 60 as shown in figure 5c to 5h.

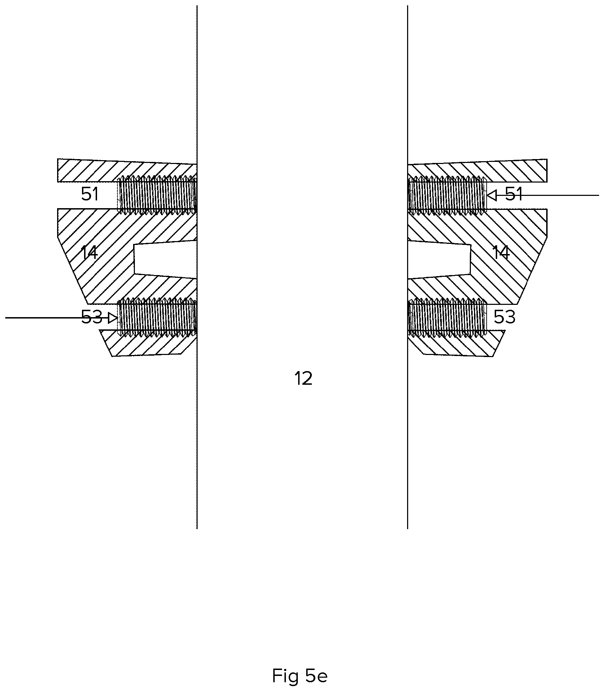

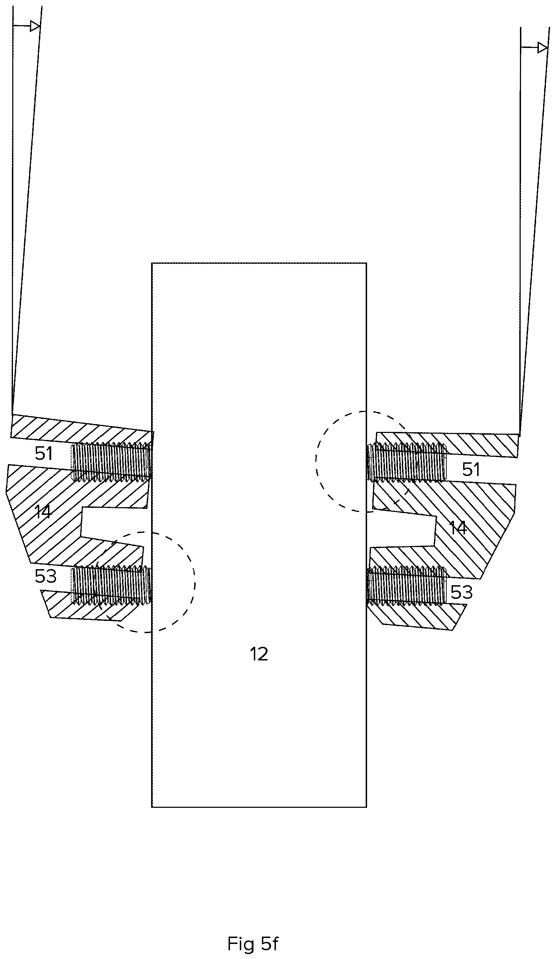

According to some aspects, the screws are used to adjust or change the angle of the cover 11 when attached to the first adapter part 13. The adjustment provides an enhanced and flexible solution for the user. The screws provide adjustments of the angle in the sagittal plane. In one aspect four holes are provided, 55, 56, 57, 58, in the second adapter part 14, as shown in 5c, where four associated screws 51, 52, 53, 54 are used for adjusting the angle. In one aspect, two screws are used for adjusting the angle, as shown in figure 5e and 5f, where figure 5e shows a side view with an unchanged angle and figure 5f shows a side view with adjusted the angle, the angel has been adjusted by adjusting at least two screws.

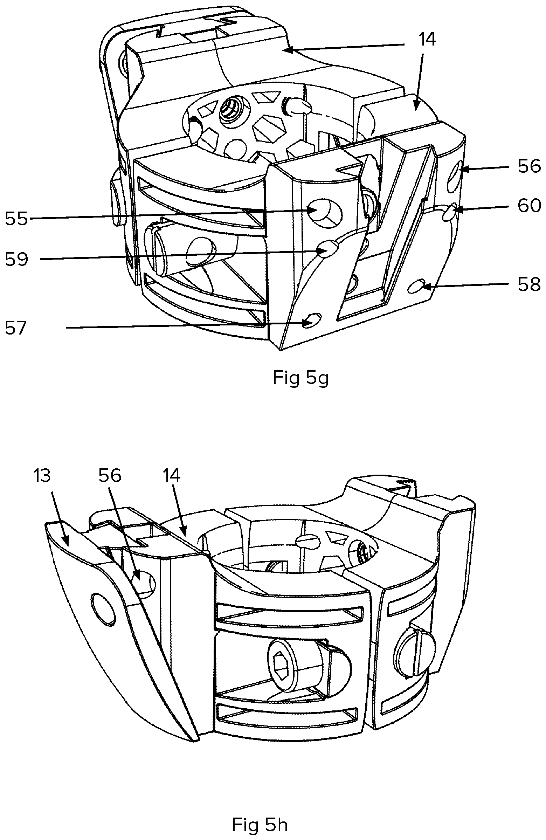

According to some aspects, at least two holes 55, 56 are provided through both the first adapter part 13 and the second adapter part 14, as shown in figure 5h, where the associated screws 51, 52 are used to fastening the first adapter part 13 onto the second adapter part 14, as shown in figure 8. The fastening aspect provides a safe and more secure solution for the user, especially for users that are active and would like to be confident with that the prosthesis will stay in place even when they are active.

Figure 5g-5h shows an example where two holes are provided through both the first adapter part 13 and the second adapter part 14, and the associated screws are used for fastening the fastening the first adapter part 13 onto the second adapter part 14. There are also provided four holes in the second adapter part 14, with associated screws (not shown), which are used for adjustment of the angle.

According to some aspects, the prosthesis system 10 further comprises at least one communication unit 111 for communicating with at least one external device 2, 3, e.g. a mobile phone 2 or a cloud service 3. In some aspect, the communication unit is configured to communicate directly 42 with at least one external device 2, such a mobile phone, or via 42, 43 a cloud service 3 or directly 41 with the cloud service 3. According to some aspects, at least one communication unit 111 is provided on one or several of the cover 11, the first adapter part 13 and/or the second adapter part 14. One example of a communication unit is pedometers that are linked to an app that gives indication to an orthopaedic engineer or user, or both, that it is time for service and maintenance of the prosthesis. Further examples of functions connected to the communication unit are wireless payment, access e.g. open locks, hotel key, bus card, distress calls e.g. emergency calls, GPS, accelerometer.

According to some aspects, the at least one cover 11 comprises a compartment 113 adapted for carrying an external device, e.g. keys, credit cards, money etc. In one example the compartment is a pocket for carrying wireless communication device, e.g. a smartphone, music player, keys etc.

A "wireless communication device" as the term may be used herein, is to be broadly interpreted to include a radiotelephone having ability for Internet/intranet access, web browser, organizer, calendar, a camera (e.g., video and/or still image camera), a sound recorder (e.g., a microphone), and/or global positioning system (GPS) receiver; a personal communications system (PCS) user equipment that may combine a cellular radiotelephone with data processing; a personal digital assistant (PDA) that can include a radiotelephone or wireless communication system; a laptop; a camera (e.g., video and/or still image camera) having communication ability; and any other computation or communication device capable of transceiving, such as a personal computer, a home entertainment system, a television, etc. Furthermore, a device may be interpreted as any number of antennas or antenna elements.

A software-based design tool, which is not part of the invention, is provided to automate and simplify the process from 3D scanning to 3D printing, thus bringing down the cost of cosmetic solutions for prostheses. The software must consider the strength of the prosthetic cover and the material cost, the existing prosthesis and interaction with it and the surrounding tissue and enable the user or designer to control the shape.

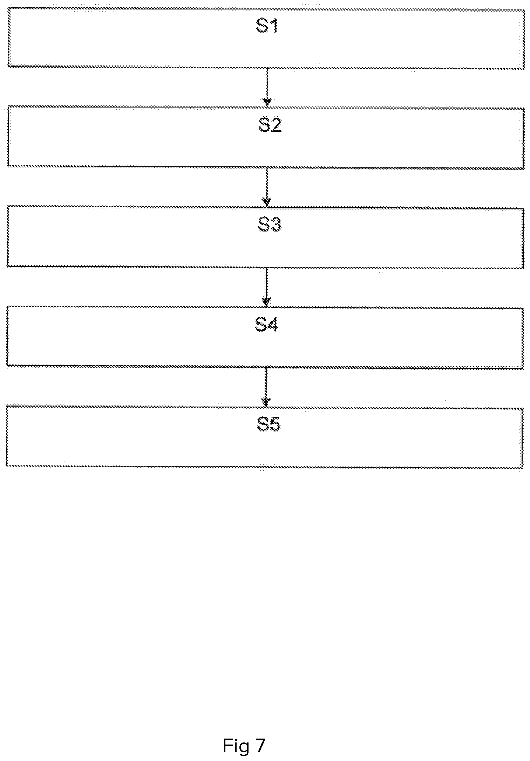

Figure 7 illustrates the steps of process for manufacturing the described prosthesis system. Firstly S1, performing a 3D scan of the geometry of the healthy limb and prosthetic limb 2 of a user. The profile of the healthy limb is traced by a CAD program. In some aspects, the healthy limb and the prosthetic limb are measured at some predetermined positions. The measurements are then provided to a program that auto generates a 3D geometry of the healthy limb.

Secondly S2, creating a reflection of the scanned geometries.

Thirdly S3, completing the design of at least one cover 11 of the prosthesis system.

As an example, when the user wear tight pants it looks like he/she has two real legs, instead of showing a glimpse of the profile of the aluminium tubes 121 which usually are the backbone of the prosthetic limb.

Fourthly S4, sending the scanned 3D file to a 3D printer for printing of the cover. The 3D printer prints the covers by using e.g. SLS technology. SLS technology is a manufacturing method that provides the best strength of a 3D printed product, such the disclosed prosthesis system.

Fifthly S5, applying a coat of paint and varnish. There are several ways to add the final surface finish, depending on what effect to achieve. Hydro dipping is a method of achieving a more graphic effect of various patterns applied to the surface via a water bath. Another way is to dress up the covers, for example, leather or textiles in order to achieve a more "dressed" effect. Additional techniques to achieve, for example, a more quality finish may be achieved by using NC milling of aluminium or wood.

According to some aspects, the communication between the communication unit 111 and the external communication device 2, 3 is encrypted. The communication between the communication unit 111 and the external communication device 2, 3 is for example encrypted using Secure Sockets Layer, SSL, or Transport Layer Security, TLS. SSL uses a combination of encryption using public keys and symmetrical keys. SSL may use several different methods of encryption, such as Data Encryption Standard, DES, Digital Signature Algorithm, DSA, Key Exchange Algorithm, KEA, Message-Digest algorithm 5, MD5, Rivest Cipher 2, RC2, Rivest Cipher4, RC4, Rivest, Shamir and Adleman encryption, RSA, Secure Hash Algorithm, SHA-1, SKIPJACK or Triple Data Encryption Standard, Triple-DES.

It should be appreciated that the operations shown in figure 7 need not be performed in order. Furthermore, it should be appreciated that not all of the operations need to be performed. Also, the functions or steps noted in the blocks can according to some aspects of the disclosure be executed continuously in a loop.

The various example embodiments described herein are described in the general context of method steps or processes, which may be implemented in one aspect by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program modules may include routines, programs, objects, components, data structures, etc. that performs particular tasks or implement particular abstract data types. Computer-executable instructions, associated data structures, and program modules represent examples of program code for executing steps of the methods disclosed herein. The particular sequence of such executable instructions or associated data structures represents examples of corresponding acts for implementing the functions described in such steps or processes.

The description of the aspects of the disclosure provided herein has been presented for purposes of illustration. The description is not intended to be exhaustive or to limit aspects of the disclosure to the precise form disclosed, and modifications and variations are possible in light of the above teachings or may be acquired from practice of various alternatives to the provided aspects of the disclosure. The examples discussed herein were chosen and described in order to explain the principles and the nature of various aspects of the disclosure and its practical application to enable one skilled in the art to utilize the aspects of the disclosure in various manners and with various modifications as are suited to the particular use contemplated. The features of the aspects of the disclosure described herein may be combined in all possible combinations of methods, apparatus, modules, systems, and computer program products. It should be appreciated that the aspects of the disclosure presented herein may be practiced in any combination with each other.

It should be noted that the word "comprising" does not necessarily exclude the presence of other elements or steps than those listed. It should further be noted that any reference signs do not limit the scope of the claims.

In the drawings and specification, there have been disclosed exemplary aspects. However, many variations and modifications can be made to these aspects. Accordingly, although specific terms are employed, they are used in a generic and descriptive sense only and not for purposes of limitation, the scope of the embodiments being defined by the following claims.

## Claims

1. An adapter device (13, 14) for fixing a cover (11) to a prosthetic limb (12) of a prosthesis system (10), comprising:
- a first adapter part (13) configured for being attached to the cover (11);
- a second adapter part (14) configured for being mounted onto the prosthetic limb (12); and
- a locking means (131, 141) for locking the first adapter part (13) onto the second adapter part (14) when the first and second adapter parts are connected;
**characterised in that**
the first adapter part is provided with at least one protrusion and the second adapter parts is provided with at least one groove for creating a dovetail connection when the first and second adapter parts are connected.

2. The adapter device (13, 14) according to claim 1, wherein the locking means comprises at least a first locking part (131) and a second locking part (141), wherein the first locking part is provided on the first adapter part (13) and the second locking part is provided on the second adapter part (14).

3. The adapter device (13, 14) according to claim 2, wherein the first locking part (131) is a resilient pressing piece and the second locking part (141) is a hole adapted for collecting the resilient pressing piece when the first and second adapter parts are connected.

4. The adapter device (13, 14) according to any of claims 1 to 3, wherein the locking means comprises magnets adapted for locking the first and second adapter part when they are connected.

5. The adapter device (13, 14) according to any of the claims 1 to 4, wherein a connecting surface (132) of the first adapter part (13) is inclined and a corresponding connecting surface (142) of the second adapter part (14) is inclined in the opposite direction according to the first adapter part (13).

6. The adapter device (13, 14) according to any of the claims 1 to 5, wherein one or both of the first adapter part (13) and the second adapter part (14) comprises a compartment adapted for enclosing a communication unit (111).

7. The adapter device (13, 14) according to any of the claims 1 to 5, wherein both of the first adapter part (13) and the second adapter part (14) are provided with at least two holes (55, 56, 57, 58, 59, 60) for engaging at least two screws (51, 52, 53, 54), wherein the at least two screws are provided for fastening the first adapter part (13) onto the second adapter part (14) and for adjusting the angle between the prosthetic limb and the adapter device.

8. The adapter device (13, 14) according claim 2, wherein the first locking part (131) is a resilient pressing piece and the second locking part (141) is a resilient pressing piece wherein the first and second adapter parts are connected and provides a double lock.

9. A prosthesis system (10) adapted for being worn by a user (1) comprising at least a first cover (11) and a prosthetic limb (12), wherein the system (10) further comprises an adapter device (13, 14) for fixing the first cover (11) to the prosthetic limb (12) according to any of claims 1-8.

10. The prosthesis system (10) according to claim 9 wherein the system further comprises at least one flexible piece (17) provided between the second adapter part (14) and the prosthetic limb (12) when attaching the second adapter part onto the prosthetic limb.

11. The prosthesis system (10) according to claim 9 or 10, wherein the system further comprises at least one communication unit (111) for communicating with at least one external device (2, 3).

12. The prosthesis system (10) according to claim 11, wherein the at least one communication unit (111) is provided on one or several of the cover (11), the first part (13) and/or the second part (14).

13. The prosthesis system (10) according to any of claims 9 to 12, wherein the at least first cover (11) comprises a compartment (113) adapted for carrying an external device.

## Patentansprüche

1. Adaptervorrichtung (13, 14) zum Fixieren einer Abdeckung (11) an einer Gliedmaßenprothese (12) eines Prothesensystems (10), umfassend:
- ein erstes Adapterteil (13), das konfiguriert ist, an der Abdeckung (11) angebracht zu werden;
- ein zweites Adapterteil (14), das konfiguriert ist, auf der Gliedmaßenprothese (12) montiert zu werden; und
- ein Verschlussmittel (131, 141) zum Verschließen des ersten Adapterteils (13) auf dem zweiten Adapterteil (14), wenn das erste und zweite Adapterteil verbunden sind;
**dadurch gekennzeichnet, dass**
das erste Adapterteil mit mindestens einem Fortsatz bereitgestellt ist und das zweite Adapterteil mit mindestens einer Kerbe bereitgestellt ist, um eine Schwalbenschwanzverbindung zu erzeugen, wenn das erste und zweite Adapterteil verbunden werden.

2. Adaptervorrichtung (13, 14) nach Anspruch 1, wobei das Verschlussmittel mindestens ein erstes Verschlussteil (131) und ein zweites Verschlussteil (141) umfasst, wobei das erste Verschlussteil an dem ersten Adapterteil (13) bereitgestellt ist und das zweite Verschlussteil an dem zweiten Adapterteil (14) bereitgestellt ist.

3. Adaptervorrichtung (13, 14) nach Anspruch 2, wobei das erste Verschlussteil (131) ein elastisches Pressstück ist und das zweite Verschlussteil (141) ein Loch ist, das zum Aufnehmen des elastischen Pressstücks angepasst ist, wenn das erste und zweite Adapterteil verbunden werden.

4. Adaptervorrichtung (13, 14) nach einem der Ansprüche 1 bis 3, wobei das Verschlussmittel Magnete umfasst, die zum Verschließen des ersten und zweiten Adapterteils angepasst sind, wenn diese verbunden werden.

5. Adaptervorrichtung (13, 14) nach einem der Ansprüche 1 bis 4, wobei eine Verbindungsoberfläche (132) des ersten Adapterteils (13) geneigt ist und eine entsprechende Verbindungsoberfläche (142) des zweiten Adapterteils (14) in die entgegengesetzte Richtung gemäß dem ersten Adapterteil (13) geneigt ist.

6. Adaptervorrichtung (13, 14) nach einem der Ansprüche 1 bis 5, wobei eines oder beide des ersten Adapterteils (13) und des zweiten Adapterteils (14) ein Fach umfasst, das angepasst ist, eine Kommunikationseinheit (111) zu umschließen.

7. Adaptervorrichtung (13, 14) nach einem der Ansprüche 1 bis 5, wobei sowohl das erste Adapterteil (13) als auch das zweite Adapterteil (14) mit mindestens zwei Löchern (55, 56, 57, 58, 59, 60) bereitgestellt sind, um mit mindestens zwei Schrauben (51, 52, 53, 54) einzugreifen, wobei die mindestens zwei Schrauben zum Befestigen des ersten Adapterteils (13) auf dem zweiten Adapterteil (14) und zum Einstellen des Winkels zwischen der Gliedmaßenprothese und der Adaptervorrichtung bereitgestellt sind.

8. Adaptervorrichtung (13, 14) nach Anspruch 2, wobei das erste Verschlussteil (131) ein elastisches Pressstück ist und das zweite Verschlussteil (141) ein elastisches Pressstück ist, wobei das erste und zweite Adapterteil verbunden sind und einen Doppelverschluss bereitstellen.

9. Prothesensystem (10), das angepasst ist, von einem Nutzer (1) getragen zu werden, umfassend mindestens eine erste Abdeckung (11) und eine Gliedmaßenprothese (12), wobei das System (10) weiter eine Adaptervorrichtung (13, 14) zum Fixieren der ersten Abdeckung (11) an der Gliedmaßenprothese (12) nach einem der Ansprüche 1-8 umfasst.

10. Prothesensystem (10) nach Anspruch 9, wobei das System weiter mindestens ein flexibles Stück (17) umfasst, das zwischen dem zweiten Adapterteil (14) und der Gliedmaßenprothese (12) bereitgestellt ist, wenn das zweite Adapterteil an der Gliedmaßenprothese angebracht wird.

11. Prothesensystem (10) nach Anspruch 9 oder 10, wobei das System weiter mindestens eine Kommunikationseinheit (111) zum Kommunizieren mit mindestens einer externen Vorrichtung (2, 3) umfasst.

12. Prothesensystem (10) nach Anspruch 11, wobei die mindestens eine Kommunikationseinheit (111) an einem oder einigen der Abdeckung (11), des ersten Teils (13) und/oder des zweiten Teils (14) bereitgestellt ist.

13. Prothesensystem (10) nach einem der Ansprüche 9 bis 12, wobei die mindestens erste Abdeckung (11) ein Fach (113) umfasst, das zum Tragen einer externen Vorrichtung angepasst ist.

## Revendications

1. Dispositif adaptateur (13, 14) pour fixer un couvercle (11) à un membre prothétique (12) d'un système de prothèse (10), comprenant :
- une première partie d'adaptateur (13) configurée pour être attachée au couvercle (11) ;
- une seconde partie d'adaptateur (14) configurée pour être montée sur le membre prothétique (12) ; et
- des moyens de verrouillage (131, 141) pour verrouiller la première partie d'adaptateur (13) sur la seconde partie d'adaptateur (14) lorsque les première et seconde parties d'adaptateur sont connectées ;
**caractérisé en ce que**
la première partie d'adaptateur est pourvue d'au moins une saillie et la seconde partie d'adaptateur est pourvue d'au moins une rainure pour créer une connexion en queue-d'aronde lorsque les première et seconde parties d'adaptateur sont connectées.

2. Dispositif adaptateur (13, 14) selon la revendication 1, dans lequel les moyens de verrouillage comprennent au moins une première partie de verrouillage (131) et une seconde partie de verrouillage (141), dans lequel la première partie de verrouillage est pourvue sur la première partie d'adaptateur (13) et la seconde partie de verrouillage est pourvue sur la seconde partie d'adaptateur (14).

3. Dispositif adaptateur (13, 14) selon la revendication 2, dans lequel la première partie de verrouillage (131) est une pièce de pression élastique et la seconde partie de verrouillage (141) est un trou adapté pour recueillir la pièce de pression élastique lorsque les première et seconde parties d'adaptateur sont connectées.

4. Dispositif adaptateur (13, 14) selon l'une quelconque des revendications 1 à 3, dans lequel les moyens de verrouillage comprennent des aimants adaptés pour verrouiller la première et la seconde partie d'adaptateur lorsqu'elles sont connectées.

5. Dispositif adaptateur (13, 14) selon l'une quelconque des revendications 1 à 4, dans lequel une surface de connexion (132) de la première partie d'adaptateur (13) est inclinée et une surface de connexion correspondante (142) de la seconde partie d'adaptateur (14) est inclinée dans la direction opposée par rapport à la première partie d'adaptateur (13).

6. Dispositif adaptateur (13, 14) selon l'une quelconque des revendications 1 à 5, dans lequel l'une ou les deux parmi la première partie d'adaptateur (13) et la seconde partie d'adaptateur (14) comprennent un compartiment adapté pour renfermer une unité de communication (111).

7. Dispositif adaptateur (13, 14) selon l'une quelconque des revendications 1 à 5, dans lequel les deux parmi la première partie d'adaptateur (13) et la seconde partie d'adaptateur (14) sont pourvues d'au moins deux trous (55, 56, 57, 58, 59, 60) pour mettre en prise au moins deux vis (51, 52, 53, 54), dans lequel les au moins deux vis sont pourvues pour fixer la première partie d'adaptateur (13) sur la seconde partie d'adaptateur (14) et pour ajuster l'angle entre le membre prothétique et le dispositif adaptateur.

8. Dispositif adaptateur (13, 14) selon la revendication 2, dans lequel la première partie de verrouillage (131) est une pièce de pression élastique et la seconde partie de verrouillage (141) est une pièce de pression élastique, dans lequel les première et seconde parties d'adaptateur sont connectées et fournissent un double verrouillage.

9. Système de prothèse (10) adapté pour être porté par un utilisateur (1) comprenant au moins un premier couvercle (11) et un membre prothétique (12), dans lequel le système (10) comprend en outre un dispositif adaptateur (13, 14) pour fixer le premier couvercle (11) au membre prothétique (12) selon l'une quelconque des revendications 1-8.

10. Système de prothèse (10) selon la revendication 9, dans lequel le système comprend en outre au moins une pièce flexible (17) pourvue entre la seconde partie d'adaptateur (14) et le membre prothétique (12) lorsque la seconde partie d'adaptateur est attachée sur le membre prothétique.

11. Système de prothèse (10) selon la revendication 9 ou 10, dans lequel le système comprend en outre au moins une unité de communication (111) pour communiquer avec au moins un dispositif externe (2, 3).

12. Système de prothèse (10) selon la revendication 11, dans lequel la au moins une unité de communication (111) est pourvue sur un ou plusieurs parmi le couvercle (11), la première partie (13) et/ou la seconde partie (14).

13. Système de prothèse (10) selon l'une quelconque des revendications 9 à 12, dans lequel le au moins un premier couvercle (11) comprend un compartiment (113) adapté pour supporter un dispositif externe.
